# EUROPÄISCHE PATENTSCHRIFT

(11) **EP 1 562 651 B1**
(45) Veröffentlichungstag und Bekanntmachung des Hinweises auf die Patenterteilung: **01.03.2006**
(21) Anmeldenummer: 03807762.4
(22) Anmeldetag: 28.07.2003
(51) Int. Cl.: A61L 27/10

(54) **KERAMISCHE ENDOPROTHESENKOMPONENTEN UND VERFAHREN ZU IHRER HERSTELLUNG**
CERAMIC ENDOPROSTHETIC ELEMENTS AND METHOD FOR THE PRODUCTION THEREOF
ELEMENTS D'ENDOPROTHESE EN CERAMIQUE ET PROCEDE DE PRODUCTION DE CES ELEMENTS

(30) Priorität: 24.09.2002 DE 10244439
(43) Veröffentlichungstag der Anmeldung: 17.08.2005
(73) Patentinhaber: Mathys AG Bettlach, 2544 Bettlach (CH)
(72) Erfinder: GLIEN, Wilfried, 07639 Bad Klosterlausnitz (DE); OBERBACH, Thomas, 07629 Reichenbach (DE); ORTMANN, Claudia, 07749 Jena (DE)
(74) Vertreter: Körfer, Thomas
(86) Internationale Anmeldenummer: PCT/EP2003/008286
(87) Internationale Veröffentlichungsnummer: WO 2004/032986

(56) Entgegenhaltungen:
- WO-A-02/11780
- DE-A- 3 806 215
- US-A- 5 232 878

## Beschreibung

Die Erfindung betrifft eine Endoprothesenkomponente aus einem keramischen Werkstoff, der im wesentlichen aus Aluminiumoxid und Zirkonium(di)oxid zusammengesetzt ist, sowie Verfahren zu ihrer Herstellung.

Aluminiumoxid und Zirkonium(di)oxid sind seit Jahren als bewährte keramische Implantatwerkstoffe bekannt. Aluminiumoxid ist sehr hart und verschleißfest. Zirkonium(di)oxid ist ein sehr bruchzähes und schadenstolerantes Material. Es ist bekannt, dass Endoprothesenkomponenten, die jeweils aus einem der beiden Materialien bestehen, in künstlichen Gelenken aufgrund der auftretenden Reibungskräfte nicht beliebig miteinander kombinierbar sind. Bewährt haben sich Paarungen von Gelenkpartnern aus Aluminiumoxid, dagegen sind Werkstoffpaarungen von Aluminiumoxid mit Zirkoniumoxid und Zirkoniumoxid mit Zirkoniumoxid in der Literatur umstritten, da es hier zu sehr starken Verschleißerscheinungen kommen kann.

EP 1 035 878 B1 stellt mögliche Werkstoffpaarungen für Gelenkpartner aus keramischen Werkstoffen vor, durch die neue konstruktive Gestaltungen mit optimalem Verschleißverhalten ermöglicht werden sollen. Die Gelenkpartner bestehen aus Sinterwerkstoffen die im wesentlichen Aluminiumoxid und Zirkoniumoxid sind, wobei mindestens einer der Gelenkpartner aus Zirkoniumoxid mit einem Zusatz von 0,1 bis 40 Gew.% Aluminiumoxid besteht. In einem Beispiel wird einem Gelenkpartner aus Zirkoniumoxid mit einem Aluminiumoxidanteil von über 5 Gew.% ein Gelenkpartner aus Aluminiumoxid zugeordnet und der Werkstoff der Kugel eines Gelenkes hat einen höheren Zirkoniumoxidanteil als der Werkstoff des keramischen Pfanneneinsatzes.

Aus WO 97/31592 ist es bekannt, bei einem künstlichen Hüftgelenk die Pfanne aus Aluminiumoxid und den Gelenkkopf aus Zirkoniumoxid herzustellen.

Aus US 2002/0031675 A1 ist eine biomedizinische Komponente bekannt, die aus 90 mol% Zirkoniumdioxid besteht, wobei das Zirkoniumdioxid durch wenigstens 2,1 mol% Yttriumoxid teilweise stabilisiert ist, und zwischen 0,05 und 1 Gew.% Aluminiumoxid enthält.

US 2002/0010070 A1 beschreibt eine biomedizinische Komponente, die aus mit Zirkoniumdioxid verstärktem Aluminiumoxid besteht, wobei 1 bis 69 Gew.% Zirkoniumoxid enthalten sind und das Zirkoniumdioxid durch mindestens 2,1 mol% Yttriumoxid oder Seltene-Erd-Oxide stabilisiert ist.

US 6 312 473 B1 beschreibt Implantatkomponenten die mit einer Schicht aus Titanium oder einer Titaniumlegierung bedeckt sind. Die offenen Poren dieser Schicht sind mit einem biokompatiblen Zement imprägniert, der durch ausgewählte Oxide, die Aluminiumoxid, Magnesiumoxid, Zirkoniumoxid oder eine Kombination dieser Oxide enthalten, verstärkt ist.

US 4 950 294 beschreibt eine Implantatkomponente mit einer Matrix aus Aluminiumoxid, Zirkonium(di)oxid und Yttriumoxid. Die Oberfläche der Matrix ist nicht monokristallin und eine bioaktive Schicht bedeckt die Matrixoberfläche.

Endoprothesenkomponenten aus einem Komposit von Aluminiumoxid und Zirkonium(di)oxid sind ferner aus EP 1 228 774 A1, EP 0 908 425 A1, JP 09268055 A und JP 11228221 A bekannt.

Aus WO 02/102275 sind Hüftgelenk-Implantate bekannt, bei denen der Gelenkkopf aus einer Metalllegierung und die Gelenkpfanne aus Zirkoniumoxid und gegebenenfalls mit Zusätzen von Aluminiumoxid besteht.

Aus DE 44 35 146 C2 ist ein Verfahren zur Herstellung eines Porositätsgradienten für Gradientenwerkstoffe bekannt, bei dem ein poröser Körper aus einem elektrisch leitenden Material in einen Elektrolyten einer Elektrolysezelle getaucht wird und durch Anwendung eines Elektrolysestroms ein anodischer Abtrag des Materials des Körpers bewirkt wird, wobei der Abtrag entlang der Verbindungslinie zwischen Anode und Kathode veränderlich ist.

Der Erfindung liegt die Aufgabe zugrunde, keramische Endoprothesenkomponenten zu schaffen, die sowohl hart und verschleißfest als auch bruchzäh und schadenstolerant sind.

Erfindungsgemäß wird diese Aufgabe dadurch gelöst, dass der Werkstoff einen Gradienten der Anteile von Aluminiumoxid und Zirkonium(di)oxid aufweist.

Dass der Werkstoff einen Gradienten der Anteile von Aluminiumoxid und Zirkonium(di)oxid aufweist bedeutet, dass sich Anteile dieser keramischen Systeme entlang des Gradienten ändern. Die erfindungsgemäße Endoprothesenkomponente hat daher keine gleichförmige Materialzusammensetzung, sondern die Aluminiumoxid- und Zirkonium(di)oxidanteile variieren innerhalb der Endoprothesenkomponente. Im Gegensatz dazu haben die Werkstoffkomposite aus Zirkoniumoxid und Aluminiumoxid, die aus den oben genannten Druckschriften bekannt sind, eine homogene Verteilung der Komponenten im Material.

Die hervorragenden Verschleißeigenschaften des Aluminiumoxids sollen vor allem in den Bereichen der Endoprothesenkomponente zum Tragen kommen, in denen keramische Gleitpartner gegeneinander artikulieren und der Werkstoff auf Reibung beansprucht wird. In diesen Bereichen ist daher der Aluminiumoxidanteil höher und kann bis zu 100% betragen. In den Bereichen der Endoprothesenkomponente, wo Spannungsmaxima und Flächenpressungen zu erwarten sind, sollen vor allem die Eigenschaften des Zirkonium(di)oxids eingebracht werden. In diesen Bereichen ist daher der Zirkonium(di)oxidanteil höher und kann bis zu 100% betragen.

Dem Aluminiumoxid können 0 bis 0,3 Gew.% Magnesiumoxid als Sinterhilfsmittel zugesetzt werden, wodurch das Kornwachstum gehemmt wird. Das Zirkonium(di)oxid kann unstabilisiert oder stabilisiert (mit den zur Phasenstabilisierung bekannten Zusätzen der Seltenen-Erd-Oxide, Erdalkalioxide, Titanoxid, Chromoxid oder Hafniumoxid) vorliegen.

Dadurch kann die Bruchfestigkeit und Bruchzähigkeit und die Schadenstoleranz der keramischen Endoprothesenkomponenten deutlich gesteigert werden. Der Werkstoff ist in der Lage Rissenergie abzubauen. Trifft ein Riss auf ein Zirkonium(di)-oxidpartikel kommt es zur Rissverzweigung und bei Vorliegen der kubischen bzw. tetragonalen Modifikation des Zirkonium-(di)oxids zu einer Phasenumwandlung in monoklines Zirkonium(di)oxid, wobei der Energieabbau erfolgt. Die Phasenumwandlung tetragonal/kubisch in monoklin ist mit einer Volumenzunahme verbunden, so dass die Rissspitze zusammengedrückt und das Risswachstum gehemmt wird. Dieser Mechanismus ist von den bisher verfügbaren umwandlungsverstärkten Keramiken bekannt.

Bei den erfindungsgemäßen Endoprothesenkomponenten handelt es sich um einen Werkstoff mit Material- und Korngrößengradient, der durch zwei verschiedene Verfahren hergestellt werden kann.

Bei dem ersten Verfahren werden in eine vorgesinterte Aluminiumoxid-Endoprothesenkomponente (Aluminiumoxidmatrix), die 0 bis 0,3 Gew.% Magnesiumoxid enthalten kann und eine hohe offene Porosität besitzt, durch Infiltration Zirkonium(di)-oxidpartikel (Partikelgröße: < 100 nm), im Falle des stabilisierten Zirkonium(di)oxids einschließlich der Stabilisatoren, eingebracht. Die Vorsinterung der Endoprothesenkomponente erfolgt bei 800 bis 1200 °C, wobei ein Schrumpfen des Werkstoffes vermieden werden muss, damit die Porosität für die Infiltration erhalten bleibt. Die Erzeugung des Gradienten im Werkstoff erfolgt mit Hilfe eines dem Schlickerguss verwandten Verfahrens. Das zirkoniumhaltige Sol (auf kolloidaler Basis bzw. polymerer Basis; H. Richter, Herstellung keramischer Nanofiltrationsmembranen aus ZrO₂ und TiO₂, Dissertation, TU Bergakademie Freiberg, Fakultät für Werkstoffwissenschaft und Werkstofftechnologie, 1999), die Zirkoniumsalzlösung, das Zirkoniumalkoholat bzw. der Zirkonium(di)oxidschlicker oder auch eine Mischung aus zwei oder mehreren der vorgenannten Lösungen/Flüssigkeiten wird (durch z.B. Gießen oder Sprühen) auf die vorgesinterte, poröse Aluminiumoxidmatrix, die bis zu 0,3 Gew.% Magnesiumoxid enthalten kann, aufgetragen bzw. sie wird infiltriert. Das zirkoniumhaltige Sol, der Zirkonium-(di)oxidschlicker, die Zirkoniumsalzlösung, das Zirkoniumalkoholat oder eine Mischung aus zwei oder mehreren der vorgenannten Lösungen/Flüssigkeiten kann die bereits erwähnten Stabilisatoren enthalten. In Abhängigkeit vom Porenvolumen, der Porengröße, der Zeit und der Konzentration des flüssigen Systems wird der porige Werkstoff bis zu einer bestimmten Tiefe infiltriert. Die Feststoffpartikel setzen sich an der inneren Oberfläche der Poren ab. Nach der Infiltration erfolgt die Trocknung der Endoprothesenkomponente, die schonend vorgenommen werden muss, damit keine Risse im Material entstehen. An diese Trocknung schließt sich ein Ausgasprozess zur Eliminierung der eventuell vorhandenen organischen Additive an. Diese organischen Additive, die im Sol, Schlicker, der Salzlösung bzw. dem Alkoholat enthalten sein können, müssen vor einer nachfolgenden Sinterung durch thermisches Ausgasen entfernt werden, da sonst Risse und Defekte im Material entstehen. Solange in der Aluminiumoxidmatrix noch genügend offene Porosität vorliegt, kann der Infiltrations-, Trocknungs- und Ausgasprozess beliebig oft wiederholt werden. Damit ist jeder gewünschte Zirkonium(di)oxidanteil in der Aluminiumoxidmatrix einstellbar. Während der anschließenden Sinterung bei 1300 bis 1600 °C entsteht ein fester, gradierter und dichter (porenfreier) Werkstoffverbund von Aluminiumoxid und Zirkonium(di)-oxid (kubische, tetragonale und monokline Phase). Auf der Seite, von der die Infiltration erfolgt, können bis zu 100 % Zirkonium(di)oxid vorliegen. Die Konzentration des Zirkonium(di)oxid nimmt von der Oberfläche in das Innere der Aluminiumoxidmatrix kontinuierlich ab. Anschließend kann eine weitere Verdichtung durch HIP (heißisostatisches Pressen) erfolgen.

Das erste beschriebene Verfahren lässt sich auch so anwenden, dass in eine unstabilisierte oder stabilisierte (mit den zur Phasenstabilisierung bekannten Zusätzen der Seltenen-Erd-Oxide, Erdalkalioxide, Titanoxid, Chromoxid oder Hafniumoxid) Zirkonium(di)oxidmatrix Aluminiumoxidpartikel mit einem Zusatz von 0 bis 0,3 Gew.% Magnesiumoxid eingelagert werden. Die einzulagernden Partikel liegen in Form von Solen, Schlicker, Salzlösungen, Alkoholaten oder Mischungen aus zwei oder mehreren der vorgenannten Lösungen/Flüssigkeiten vor.

Die Matrix, die infiltriert wird, kann auch ein homogener poröser Komposit sein. Die Matrix kann also Aluminiumoxid, Zirkon(di)oxid oder ein Komposit sein. Der Komposit kann zwischen 0-100% aus Aluminiumoxid und entsprechend dazu zwischen 100-0% Zirkon(di)oxid bestehen. Bei dem Zirkon(di)-oxid kann es sich um ein unstabilisiertes oder ein mit den üblichen Stabilisatoren (seltene Erdoxide, Erdalkalioxide, Titanoxid, Chromoxid oder Hafniumoxid - die als Phasenstabilisatoren bekannt sind) stabilisiertes Zirkon(di)oxid handeln.

Bei dem zweiten Herstellungsverfahren wird der Gradient im Werkstoff über uniaxiales oder isostatisches Trockenpressen hergestellt. Die Pressform wird kontinuierlich mit Zirkonium-(di)oxid- und Aluminiumoxidpulver gefüllt, wobei das Mischverhältnis von Zirkonium(di)oxid und Aluminiumoxid mit bis zu 0,3 Gew.% Magnesiumoxid je nach Erfordernis bzw. Design der Endoprothesenkomponente kontinuierlich variiert wird. Durch anschließendes Pressen wird ein Formkörper hergestellt. Alle Mischungsverhältnisse der Hauptkomponenten Zirkonium(di)oxid (stabilisiert und unstabilisiert) und Aluminiumoxid (zwischen 100 % Zirkonium(di)oxid und 0 % Aluminiumoxid und 100 % Aluminiumoxid und 0 % Zirkonium(di)oxid) sind realisierbar. Bereiche der Keramikkomponente können auch nur aus Aluminiumoxid, nur aus Zirkonium(di)oxid oder aus einem Komposit aus Aluminiumoxid und Zirkon(di)oxid homogener Zusammensetzung bestehen, wobei dann andere Bereiche aus einem gradierten Material bestehen. Bei dem Zirkon(di)oxid kann es sich wiederum um unstabilisiertes oder stabilisiertes Material handeln.

Nach der Formgebung erfolgt eine thermische Behandlung in zwei Schritten. Im ersten Schritt wird die noch in den Formkörpern vorhandene Organik ausgegast und im zweiten Schritt erfolgt die Sinterung der Formkörper bei 1300 bis 1600 °C zur Erzielung eines festen, gradierten und dichten Werkstoffverbundes von Aluminiumoxid und Zirkonium(di)oxid (monokline, tetragonale, kubische Phase). Auch hier kann der Formkörper durch HIP weiter verdichtet werden.

Es besteht auch die Mögliahkeit, dass der hergestellte Formkörper nach dem Ausgasen vor oder nach einem eventuellen Vorsintern einem Infiltrationsverfahren, entsprechend dem oben beschriebenen ersten Verfahren, unterzogen wird.

Bei beiden Herstellungsverfahren gehen beide Phasen (Aluminiumoxid und Zirkonium(di)oxid) jeweils homogen ineinander über. Das Zirkonium(di)oxid liegt in tetragonaler, kubischer oder monokliner Modifikation vor.

Ausführungsbeispiele der erfindungsgemäßen keramischen Endoprothesenkomponenten und das Prinzip der Gradientenerzeugung im Werkstoff sind in den Zeichnungen dargestellt und werden im folgenden näher beschrieben. Es zeigen:
- Fig. 1: einen Hüftgelenkkopf aus Keramik im Schnitt,
- Fig. 2: eine Keramikpfanne eines künstlichen Gelenkes im Schnitt,
- Fig. 3: ein Keramikinlay eines künstlichen Gelenkes im Schnitt,
- Fig. 4: eine keramische Kondylenkufe eines künstlichen Kniegelenkes und
- Fig. 5 bis 7: das Prinzip der Gradientenerzeugung im Werkstoff.

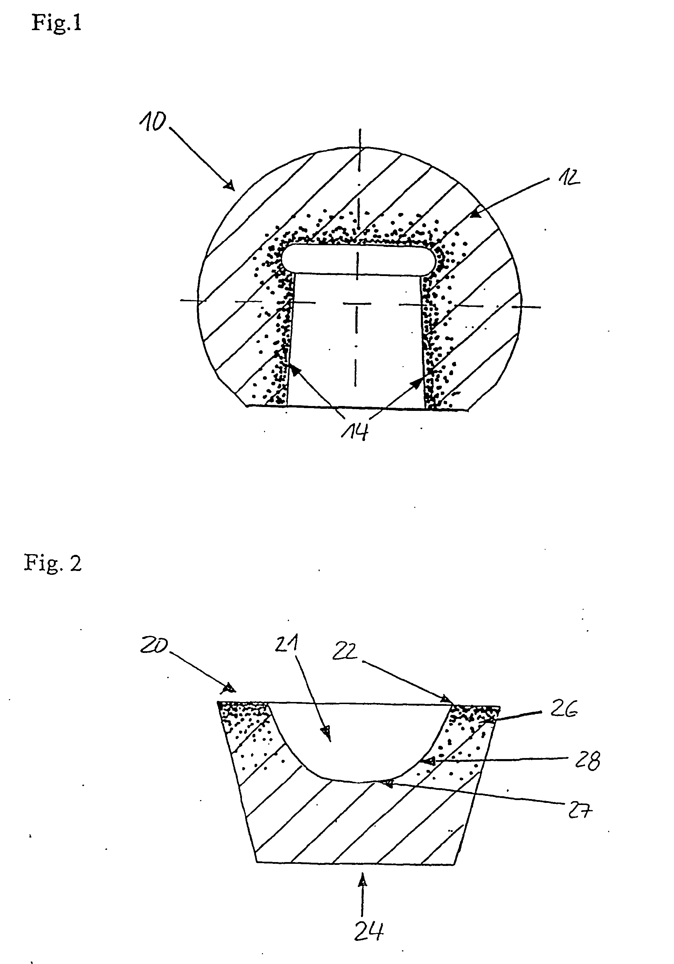

Fig. 1 zeigt einen auf einen Femurschaft aufsteckbaren Hüftgelenkkopf 10 aus Keramik, der dafür vorgesehen ist, gegen eine Hüftgelenkpfanne oder ein Inlay in einer künstlichen Hüftgelenkpfanne zu artikulieren. Der Gelenkkopf 10 besteht aus Aluminiumoxid und Zirkonium(di)oxid, wobei der Werkstoff einen Konzentrationsgradienten aufweist und sich die Anteile von Aluminiumoxid und Zirkonium(di)oxid entlang des Gradienten ändern. Die Oberfläche 12 des Gelenkkopfes 10 ist durch Reibung beansprucht und enthält daher einen erhöhten Aluminiumoxidanteil. Die Oberfläche 12 besteht aus 100 % Aluminiumoxid. Der Hüftgelenkkopf 10 hat einen Innenkonus 14, mit dem er auf den Femurschaft aufgesteckt wird. Im Innenkonus 14 des Gelenkkopfes 10 liegen nach dem Aufstecken des Hüftkopfes 10 auf den Schaftkegel hohe Flächenpressungen und Spannungen in Umfangsrichtung vor. Es können schlag- und stoßartige Beanspruchungen auftreten. Ein hoher Anteil an Zirkonium(di)oxid führt hier zu einer erhöhten Bruchfestigkeit, Bruchzähigkeit und Schadenstoleranz, als es beim reinen Aluminiumoxid der Fall ist. Die Oberfläche des Innenkonus 14 besteht aus nahezu 100 % Zirkonium (di) oxid.

Die Herstellung des Gelenkkopfes 10 mit einem Gradienten im Werkstoff erfolgt gemäß dem oben beschriebenen ersten Verfahren: Zunächst wird ein Aluminiumoxid-Gelenkkopf 10 hergestellt und bei 800 bis 1300 °C vorgesintert, so dass ein Schrumpfen des Werkstoffes vermieden wird, damit die Porosität für die Infiltration erhalten bleibt. Die Aluminiumoxidmatrix enthält 0,3 Gew.% Magnesiumoxid und besitzt eine hohe offene Porosität. Der Zirkonium(di)oxidschlicker, das zirkoniumhaltige Sol, die Zirkoniumsalzlösung, das Zirkoniumalkoholat oder Mischungen aus zwei oder mehreren der vorgenannten Lösungen/Flüssigkeiten werden in den Innenkonusbereiche 14 gegossen. Durch Infiltration wird zirkoniumhaltiges Material einschließlich der Stabilisatoren in die porösen Bereiche der Aluminiumoxidmatrix eingebracht. In Abhängigkeit vom Porenvolumen, der Porengröße, der Zeit und der Konzentration des flüssigen Systems wird der porige Gelenkkopf 10 bis zur gewünschten Tiefe von 5 mm infiltriert. Die Feststoffpartikel mit den Stabilisatoren setzten sich an der inneren Oberfläche der Poren ab. Nach der Infiltration erfolgt die Trocknung des Gelenkkopfes 10, die schonend vorgenommen werden muss, damit keine Risse im Material entstehen. An diese Trocknung schließt sich ein Ausgasprozess zur Eliminierung der eventuell vorhandenen organischen Additive an. Die Infiltration, Trocknung und Ausgasung kann mehrmals wiederholt werden, so dass sich auf der Innenfläche des Konus 14 ein Zirkonium(di)oxidanteil von nahezu 100 % ergibt. Während der anschließenden Sinterung bei 1300 bis 1600 °C entsteht ein fester, gradierter und dichter Werkstoffverbund von Aluminiumoxid und Zirkonium(di)oxid (kubische, tetragonale und monokline Phase). Anschließend erfolgt eine weitere Verdichtung des Gelenkkopfes 10 durch HIP (heißisostatisches Pressen).

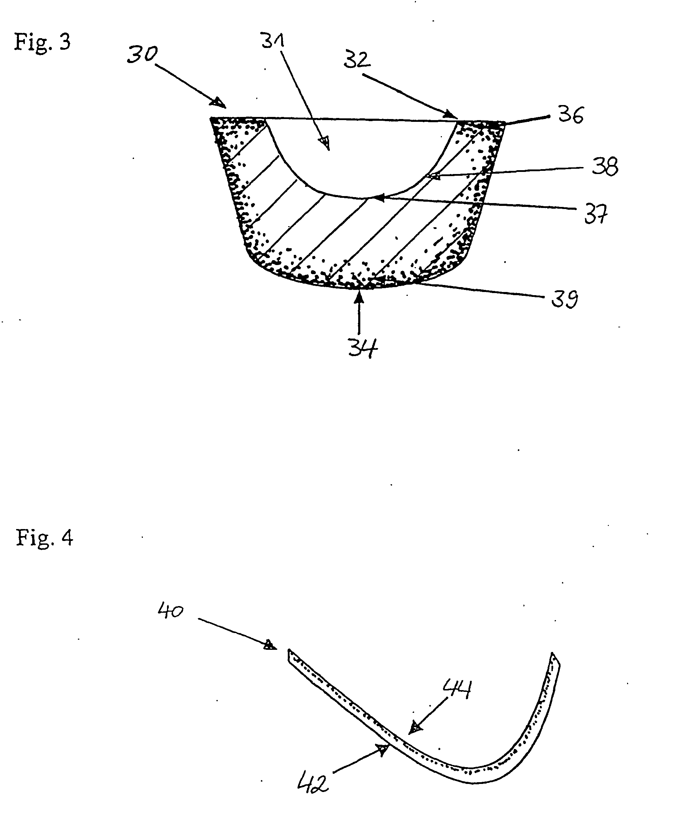

Fig. 2 zeigt eine Keramikpfanne 20 und Fig. 3 zeigt ein Keramikinlay 30, die dafür vorgesehen sind, dass gegen die Innenseiten 21, 31 ein Gelenkkopf, z.B. der in Fig. 1 beschriebene Hüftgelenkkopf 10, artikuliert. Keramikpfanne 20 und Keramikinlay 30 besitzen jeweils eine Stirnfläche und Kalottenrand 22, 32 und eine Unterseite 24, 34. Bei der Keramikpfanne 20 weist der obere Bereich 26 zur Realisierung einer hohen Schadenstoleranz im Falle von Impingement (Anschlagen des Schafthalses während der Bewegung), Subluxation und Randläufern des Keramikkopfes einen Zirkonium(di)oxid-anteil von nahezu 100 % auf. Im Bereich des Pols 27 der Kalotte und im Bereich der Hauptartikulation 28 zwischen Keramikkopf und Kalotte beträgt der Aluminiumoxidanteil 100 %, um eine hohe Verschleißfestigkeit zu gewährleisten. Das Keramikinlay 30 wird im oberen Bereich 36 wie die Keramikpfanne 20 beansprucht und weist daher in diesem Bereich 36 einen Zirkonium(di)oxidanteil von nahezu 100 % auf. Zusätzlich unterliegt die Unterseite 34 des Inlays 30 Zugspannungen, da das Inlay 30 in einer metallischen Schraubpfanne durch konische Klemmung befestigt wird. Daher ist im unteren Bereich 39 ein Zirkonium(di)oxidanteil von nahezu 100 % realisiert. Der Zirkonium(di)oxidanteil nimmt zum Pol 37 der Kalotte und zum Artikulationsbereich 38 hin kontinuierlich ab, da hier besonders gute Verschleißeigenschaften erforderlich sind, die durch einen Aluminiumoxidanteil von 100 % gewährleistet werden.

Die Herstellung der Keramikpfanne 20 und des Gradienten im Werkstoff erfolgt gemäß dem oben beschriebenen zweiten Verfahren: Eine zur Herstellung einer Keramikpfanne 20 erforderliche Pressform wird kontinuierlich mit Aluminiumoxid- und Zirkonium(di)oxidpulver gefüllt, wobei das Mischungsverhältnis von Zirkonium(di)oxid und Aluminiumoxid mit 0,3 Gew.% Magnesiumoxid kontinuierlich variiert wird. Das Befüllen der Pressform beginnt in den Bereichen, die später die Unterseite 24 der Pfanne bilden und setzt sich in Richtung Stirnfläche und Kalottenrand 22 fort. Bis auf die Höhe des späteren Hauptartikulationsbereiches 28 der Kalotte wird die Pressform mit Aluminiumoxidpulver gefüllt. Beim weiteren Befüllen in Richtung Stirnfläche und Kalottenrand 22 wird dann ein Pulvergemisch aus Aluminiumoxid und Zirkonium(di)oxid verwendet, wobei der Anteil des Zirkonium(di)oxidpulvers kontinuierlich zunimmt und im oberen Bereich 26 der Keramikpfanne 20 dann nahezu 100 % beträgt. Durch uniaxiales Trockenpressen und nachfolgende Grünbearbeitung wird unter Zugabe organischer Binder die Keramikpfanne 20 hergestellt. Nach der Formgebung erfolgt eine thermische Behandlung der Keramikpfanne 20 in zwei Schritten. Im ersten Schritt wird die noch vorhandene Organik ausgegast und im zweiten Schritt erfolgt die Sinterung bei 1300 bis 1600 °C zur Erzielung eines festen, gradierten und dichten Werkstoffverbundes von Aluminiumoxid und Zirkonium(di)oxid (monokline, tetragonale, kubische Phase), der anschließend durch HIP weiter verdichtet werden kann.

Die Herstellung des Keramikinlays 30 und des Gradienten im Werkstoff erfolgt gemäß dem oben beschriebenen ersten Verfahren: Zunächst wird nach dem gleichen Prinzip wie beim Gelenkkopf in Fig. 1 ein vorgesintertes Inlay aus Aluminiumoxid hergestellt. Danach wird durch Infiltration mit Zirkonium(di)oxid einschließlich der bekannten Stabilisatoren im Werkstoff ein Gradient erzeugt. Dazu wird das Inlay 30 in ein zirkoniumhaltiges Sol, eine Zirkoniumoxidsalzlösung, ein Zirkoniumalkoholat, einen Zirkonoxidschlicker oder eine Mischung der vorgenannten Lösungen/Flüssigkeiten eingetaucht, so dass die gesamte äußere Oberfläche mit Ausnahme der Innenseitenbereiche 31, die für die Artikulation mit einem Gelenkkopf vorgesehen sind, bedeckt ist. Die vorgesinterte, poröse Aluminiumoxidmatrix kann bis zu einer Tiefe von etwa 5 mm infiltriert werden. Nach der Infiltration erfolgt die Trocknung und Ausgasung. Die Infiltration, Trocknung und Ausgasung kann beliebig oft wiederholt werden, so dass sich auf der gesamten äußeren Oberfläche ein Zirkonium(di)oxidanteil von nahezu 100 % ergibt. Anschließend erfolgt die Sinterung bei 1300 bis 1600 °C. Eine weitere Verdichtung durch HIP ist möglich.

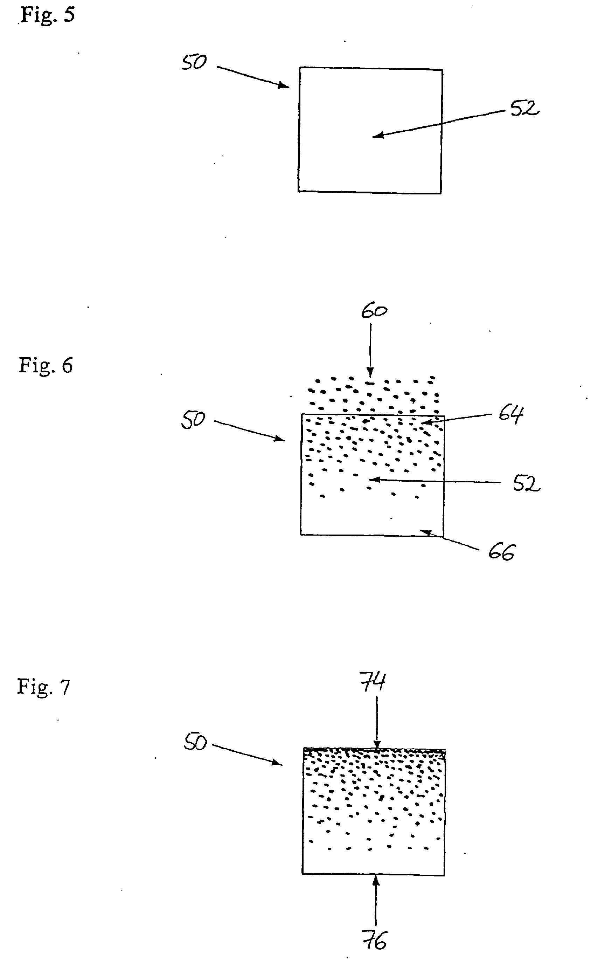

Fig. 4 zeigt eine keramische Kondylenkufe 40 eines künstlichen Kniegelenkes, die aus Aluminiumoxid und Zirkonium(di)oxid besteht und deren Anteile sich entlang eines Gradienten ändern. Durch das Einbringen von Zirkonium(di)oxid wird die Bruchzähigkeit und Schadenstoleranz der gesamten Kufe 40 erhöht. Die auf Reibung und Verschleiß beanspruchte Artikulationsfläche (Außenseite) 42 besteht aus 100% Aluminiumoxid während die Innenseite 44 der Kufe zum Abbau der Steifigkeit und zur Erhöhung der Bruchzähigkeit und Schadenstoleranz aus nahezu 100 % Zirkonium(di)oxid besteht.

Die Herstellung der Kondylenkufe 40 und des Gradienten im Werkstoff erfolgt gemäß dem oben beschriebenen ersten Verfahren: Zuerst wird eine Kondylenkufe 40 aus Aluminiumoxid hergestellt und bei 800 bis 1300 °C vorgesintert. Danach wird auf die Innenseite 44 der Kufe 40 ein Zirkonium(di)oxidschlicker, ein Zirkoniumalkoholat, ein zirkoniumhaltiges Sol bzw. eine Zirkoniumsalzlösung oder eine Mischung aus zwei oder mehreren der vorgenannten Lösungen/Flüssigkeiten aufgebracht und die vorgesinterte, poröse Aluminiumoxidmatrix wird von dieser Seite aus infiltriert. Nach der Infiltration erfolgt eine Trocknung und Ausgasung. Auf der gesamten Innenseite 44 ergibt sich ein Zirkonium(di)oxidanteil von nahezu 100 %, der in Richtung Artikulationsfläche (Außenseite) 42 kontinuierlich abnimmt. Auf der Außenseite 42 besteht die Kondylenkufe 40 aus 100 % Aluminiumoxid. Die Kondylenkufe 40 wird bei 1300 bis 1600 °C gesintert und kann anschließend durch HIP verdichtet werden.

Die Figuren 5 bis 7 zeigen schematisch die Infiltration einer Aluminiumoxidmatrix mit zirkoniumhaltigem Material zur Erzeugung eines Gradienten im Werkstoff. Eine vorgesinterte Aluminiumoxid-Endoprothesenkomponente 50 besteht aus einer Aluminiumoxidmatrix 52 mit hoher offener Porosität, die 0 bis 0,3 Gew.% Magnesiumoxid enthalten kann (Fig. 5). Durch Infiltration wird zirkoniumhaltiges Material 60 (Partikelgröße: < 100 nm), im Falle des stabilisierten Zirkonium(di)oxid einschließlich der Stabilisatoren; in die Matrix 52 eingebracht (Fig. 6). In Abhängigkeit vom Porenvolumen, der Porengröße, der Zeit und der Konzentration des flüssigen Systems wird der porige Werkstoff bis zu einer bestimmten Tiefe infiltriert. Die zirkoniumhaltigen Partikel setzten sich an der inneren Oberfläche der Poren ab und die Konzentration des Zirkonium(di)oxid nimmt von der Oberfläche 64 in das Innere 66 der Aluminiumoxidmatrix kontinuierlich ab. Auf der Seite 74, von der die Infiltration erfolgt, können bis zu 100 % Zirkonium(di) oxid und auf der gegenüberliegenden Seite 76 bis zu 100 % Aluminiumoxid vorliegen (Fig. 7).

## Patentansprüche

1. Endoprothesenkomponente aus einem keramischen Werkstoff, der Aluminiumoxid und Zirkonium(di)oxid enthält, wobei das Zirkonium(di)oxid unstabilisiert oder stabilisiert (mit den zur Phasenstabilisierung bekannten Zusätzen der Seltenen-Erd-Oxide, Erdalkalioxide, Titanoxid, Chromoxid oder Hafniumoxid) vorliegt,
**dadurch gekennzeichnet, dass** der Werkstoff einen Gradienten der Anteile von Aluminiumoxid und Zirkonium(di)oxid aufweist.

2. Endoprothesenkomponente nach Anspruch 1, die Teil eines künstlichen Gelenkes ist, einen Artikulationsbereich hat und einen Bereich hat, der im Einsatzfall erhöhten Zug-, Biege- und Torsionsspannungen unterliegt, **dadurch gekennzeichnet, dass** der Bereich erhöhter Zug-, Biege- und Torsionsspannungen einen im Vergleich zum Artikulationsbereich erhöhten Anteil an Zirkonium(di)oxid und der Artikulationsbereich vorrangig Aluminiumoxid enthält.

3. Endoprothesenkomponente nach Anspruch 1 oder 2, **dadurch gekennzeichnet, dass** zwei Bereiche vorliegen und in einem Bereich der Werkstoff ein Mischungsverhältnis zwischen nahezu 100 % Aluminiumoxid und 0 % Zirkonium(di)oxid und im anderen Bereich ein Mischungsverhältnis von 0 % Aluminiumoxid und nahezu 100 % Zirkonium(di)oxid hat.

4. Endoprothesenkomponente nach einem der Ansprüche 1 bis 3, **dadurch gekennzeichnet, dass** im Werkstoff ein Konzentrationsgradient von Aluminiumoxid und Zirkonium(di)oxid vorliegt.

5. Endoprothesenkomponente nach einem der Ansprüche 1 bis 4, **dadurch gekennzeichnet, dass** im Werkstoff ein Korngrößengradient vorliegt, der an den Konzentrationsgradienten gekoppelt ist.

6. Endoprothesenkomponente nach einem der Ansprüche 1 bis 5, **dadurch gekennzeichnet, dass** das Aluminiumoxid einen Anteil von 0 bis 0,3 Gew.% Magnesiumoxid aufweist.

7. Verfahren zur Herstellung einer Endoprothesenkomponente nach einem der Ansprüche 1 bis 6, **dadurch gekennzeichnet, dass** die Endoprothesenkomponente aus einer offenporigen vorgesinterten Aluminiumoxidmatrix mit einem Zirkonium(di)oxidschlicker, einer Zirkoniumsalzlösung, einem zirkoniumhaltigen Sol oder Alkoholat oder einer Mischung aus zwei oder mehr der vorgenannten Lösungen/Flüssigkeiten infiltriert wird, wobei Teilchen im Größenbereich von < 100 nm in die offenporige Aluminiumoxidmatrix eingebracht werden.

8. Verfahren zur Herstellung einer Endoprothesenkomponente nach einem der Ansprüche 1 bis 6, **dadurch gekennzeichnet, dass** die Endoprothesenkomponente aus einer offenporigen vorgesinterten Zirkonium(di)oxidmatrix (stabilisiert mit den zur Phasenstabilisierung bekannten Zusätzen der Seltenen-Erd-Oxide, Erdalkalioxide, Titanoxid, Chromoxid oder Hafniumoxid oder unstabilisiert) mit Aluminiumoxidpartikeln mit 0 bis 0,3 Gew.% Magnesiumoxid infiltriert wird, wobei Teilchen im Größenbereich von < 100 nm in die offenporige Zirkonium(di)oxidmatrix eingebracht werden

9. Verfahren nach Anspruch 7 oder 8, wobei die Endoprothesenkomponente eine Matrix in Form eines homogenen, porösen Komposits aus Zirkon(di)oxid und Aluminiumoxid aufweist.

10. Verfahren zur Herstellung einer Endoprothesenkomponente nach Anspruch 7 oder 9, **dadurch gekennzeichnet, dass** die Infiltration durch z.B. Sprühen, Tauchen oder Gießen erfolgt.

11. Verfahren zur Herstellung einer Endoprothesenkomponente nach einem der Ansprüche 7 bis 10, **dadurch gekennzeichnet, dass** die infiltrierte Matrix der Endoprothesenkomponente nach der Infiltration einem Trocknungs-, Ausgas- und Sinterungsprozess unterzogen wird.

12. Verfahren zur Herstellung einer Endoprothesenkomponente nach einem der Ansprüche 7 bis 11, **dadurch gekennzeichnet, dass** der Infiltrations-, Trocknungs- und Ausgasprozess zur Erhöhung des Zirkonium(di)oxidanteils in der Matrix mehrfach wiederholt wird.

13. Verfahren zur Herstellung einer Endoprothesenkomponente nach einem der Ansprüche 1 bis 6, **dadurch gekennzeichnet, dass** eine Pressform zumindest teilweise kontinuierlich mit einer Mischung aus Aluminiumoxid- und Zirkonium(di)oxid-pulver gefüllt wird, wobei während des Befüllens der Pressform das Mischverhältnis von Aluminiumoxid und Zirkonium (di) oxid variiert wird, und dass durch Pressen ein Formkörper hergestellt wird.

14. Verfahren zur Herstellung einer keramischen Endoprothesenkomponente nach Anspruch 13, **dadurch gekennzeichnet, dass** das Pressen uniaxial oder isostatisch durchgeführt wird.

15. Verfahren zur Herstellung einer Endoprothesenkomponente nach Anspruch 13 oder 14, **dadurch gekennzeichnet, dass** der gepresste Formkörper ausgegast und gesintert wird.

16. Verfahren zur Herstellung einer Endoprothesenkomponente nach Anspruch 13 oder 14 **dadurch gekennzeichnet, dass** der hergestellte Formkörper ausgegast wird und anschließend einem Infiltrationsverfahren - wie in den Ansprüchen 7 bis 11 beschrieben - unterzogen wird.

17. Verfahren zur Herstellung einer Endoprothesenkomponente nach einem der Ansprüche 7 bis 16, **dadurch gekennzeichnet, dass** eine weitere Verdichtung des gesinterten Formkörpers durch heißisostatisches Pressen erfolgt.

## Claims

1. Endoprosthetic component made of a ceramic material containing aluminium oxide and zirconium (di)oxide, the zirconium (di)oxide being unstabilized or stabilized (with the additives known for phase stabilization, i.e. rare earth oxides, alkaline earth metal oxides, titanium oxide, chromium oxide or hafnium oxide), **characterized in that** the proportions of aluminium oxide and zirconium (di)oxide in the material are in a gradient.

2. Endoprosthetic component according to Claim 1 which is part of an artificial joint and has an articulation region and a region that is subject to increased tensile, flexural and torsional stresses when in use, **characterized in that** the region of increased tensile, flexural and torsional stresses contains a greater proportion of zirconium (di)oxide than the articulation region, the latter containing predominantly aluminium oxide.

3. Endoprosthetic component according to Claim 1 or 2, **characterized in that** there are two regions, the material in one region having a mixing ratio of almost 100% aluminium oxide and 0% zirconium (di)oxide and the material in the other region having a mixing ratio of 0% aluminium oxide and almost 100% zirconium (di)oxide.

4. Endoprosthetic component according to one of Claims 1 to 3, **characterized in that** there is a concentration gradient of aluminium oxide and zirconium (di)oxide in the material.

5. Endoprosthetic component according to one of Claims 1 to 4, **characterized in that** there is a particle size gradient in the material which is linked to the concentration gradient.

6. Endoprosthetic component according to one of Claims 1 to 5, **characterized in that** the aluminium oxide has a magnesium oxide content of 0 to 0.3 wt.%.

7. Process for the production of an endoprosthetic component according to one of Claims 1 to 6, **characterized in that** the endoprosthetic component, consisting of an open-pore presintered aluminium oxide matrix, is infiltrated with a zirconium (di)oxide slip, a zirconium salt solution, a zirconium-containing sol or alcoholate, or a mixture of two or more of the aforementioned solutions/liquids, particles in the size range of <100 nm being introduced into the open-pore aluminium oxide matrix.

8. Process for the production of an endoprosthetic component according to one of Claims 1 to 6, **characterized in that** the endoprosthetic component, consisting of an open-pore presintered zirconium (di)oxide matrix (stabilized with the additives known for phase stabilization, i.e. rare earth oxides, alkaline earth metal oxides, titanium oxide, chromium oxide or hafnium oxide, or unstabilized), is infiltrated with aluminium oxide particles containing 0 to 0.3 wt.% of magnesium oxide, particles in the size range of <100 nm being introduced into the open-pore zirconium (di)oxide matrix.

9. Process according to Claim 7 or 8 wherein the endoprosthetic component has a matrix in the form of a homogeneous porous composite of zirconium (di)oxide and aluminium oxide.

10. Process for the production of an endoprosthetic component according to Claim 7 or 9, **characterized in that** the infiltration is effected e.g. by spraying, dipping or pouring.

11. Process for the production of an endoprosthetic component according to one of Claims 7 to 10, **characterized in that** the infiltrated matrix of the endoprosthetic component is subjected to a drying, outgassing and sintering process after infiltration.

12. Process for the production of an endoprosthetic component according to one of Claims 7 to 11, **characterized in that** the infiltration, drying and outgassing process is repeated several times to increase the proportion of zirconium (di)oxide in the matrix.

13. Process for the production of an endoprosthetic component according to one of Claims 1 to 6, **characterized in that** a mould is at least partially filled continuously with a mixture of aluminium oxide and zirconium (di)oxide powder, the mixing ratio of aluminium oxide and zirconium (di)oxide being varied during the filling of the mould, and **in that** a moulding is produced by pressing.

14. Process for the production of a ceramic endoprosthetic component according to Claim 13, **characterized in that** the pressing is carried out uniaxially or isostatically.

15. Process for the production of an endoprosthetic component according to Claim 13 or 14, **characterized in that** the pressed moulding is outgassed and sintered.

16. Process for the production of an endoprosthetic component according to Claim 13 or 14, **characterized in that** the moulding produced is outgassed and then subjected to an infiltration process as described in Claims 7 to 11.

17. Process for the production of an endoprosthetic component according to one of Claims 7 to 16, **characterized in that** a further densification of the sintered moulding is effected by hot isostatic pressing.

## Revendications

1. Composant d'endoprothèse en matériau céramique, qui contient de l'oxyde d'aluminium et du (di)oxyde de zirconium, le (di)oxyde de zirconium étant présent de façon non stabilisée ou stabilisée (avec les quantités ajoutées connues pour la stabilisation de phase d'oxydes de terres rares, d'oxydes alcalino-terreux, d'oxyde de titane, d'oxyde de chrome ou d'oxyde de hafnium),
**caractérisé en ce que** le matériau comprend un gradient des teneurs en oxyde d'aluminium et (di)oxyde de zirconium.

2. Composant d'endoprothèse selon la revendication 1, qui fait partie d'une articulation artificielle, présente une zone d'articulation et une zone qui est soumise dans le cas utilisé à des contraintes de traction, de flexion et de torsion plus élevées, **caractérisé en ce que** la zone soumise à des contraintes de traction, de flexion et de torsion plus élevées contient une teneur en (di)oxyde de zirconium plus élevée par comparaison de la zone d'articulation et la zone d'articulation contient en priorité de l'oxyde d'aluminium.

3. Composant d'endoprothèse selon la revendication 1 ou 2, **caractérisé en ce qu'**il existe deux zones et dans une zone, le matériau présente un rapport de mélange compris entre presque 100% d'oxyde d'aluminium et 0% de (di)oxyde de zirconium et dans l'autre zone un rapport de mélange de 0% d'oxyde d'aluminium et de presque 100% de (di)oxyde de zirconium.

4. Composant d'endoprothèse selon l'une quelconque des revendications 1 à 3, **caractérisé en ce qu'**un gradient de concentration d'oxyde d'aluminium et de (di)oxyde de zirconium est présent dans le matériau.

5. Composant d'endoprothèse selon l'une quelconque des revendications 1 à 4, **caractérisé en ce qu'**un gradient de granulométrie, qui est couplé au gradient de concentration, est présent dans le matériau.

6. Composant d'endoprothèse selon l'une quelconque des revendications 1 à 5, **caractérisé en ce que** l'oxyde d'aluminium comprend une teneur de 0 à 0,3% en poids d'oxyde de magnésium.

7. Procédé de fabrication d'un composant d'endoprothèse selon l'une quelconque des revendications 1 à 6, **caractérisé en ce que** le composant d'endoprothèse est infiltré à partir d'une matrice d'oxyde d'aluminium préfrittée à pores ouverts avec une suspension de (di)oxyde de zirconium, une solution saline de zirconium, un sol ou un alcoolat à teneur en zirconium ou un mélange de deux ou plus des solutions/fluides cités, les particules dans la granulométrie <100 nm étant introduites dans la matrice d'oxyde d'aluminium à pores ouverts.

8. Procédé de fabrication d'un composant d'endoprothèse selon l'une quelconque des revendications 1 à 6, **caractérisé en ce que** le composant d'endoprothèse est infiltré à partir d'une matrice de (di)oxyde de zirconium préfrittée à pores ouverts (stabilisée avec les quantités ajoutées connues pour la phase de stabilisation d'oxydes de terres rares, d'oxydes alcalino-terreux, d'oxyde de titane, d'oxyde de chrome ou d'oxyde de hafnium ou non stabilisée) avec des particules d'oxyde d'aluminium présentant 0 à 0,3% en poids d'oxyde de magnésium, les particules dans la granulométrie <100 nm étant introduites dans la matrice de (di)oxyde de zirconium à pores ouverts.

9. Procédé selon la revendication 7 ou 8, le composant d'endoprothèse comprenant une matrice sous forme d'un composite homogène, poreux en (di)oxyde de zirconium et oxyde d'aluminium.

10. Procédé de fabrication d'un composant de prothèse selon la revendication 7 ou 9, **caractérisé en ce que** l'infiltration s'effectue par exemple par pulvérisation, immersion ou coulée.

11. Procédé de fabrication d'un composant de prothèse selon l'une quelconque des revendications 7 à 10, **caractérisé en ce que** la matrice infiltrée du composant d'endoprothèse est soumise après l'infiltration à un processus de séchage, de dégazage et de frittage.

12. Procédé de fabrication d'un composant d'endoprothèse selon l'une quelconque des revendications 7 à 11, **caractérisé en ce que** le processus d'infiltration, de séchage et de dégazage est répété plusieurs fois pour augmenter la quantité de (di)oxyde de zirconium dans la matrice.

13. Procédé de fabrication d'un composant d'endoprothèse selon l'une quelconque des revendications 1 à 6, **caractérisé en ce qu'**un moule de pressage est rempli au moins en partie en continu d'un mélange de poudre d'oxyde d'aluminium et de (di)oxyde de zirconium, le rapport de mélange de l'oxyde d'aluminium et du (di)oxyde de zirconium variant pendant le remplissage du moule de pressage, et **en ce qu'**un corps moulé est fabriqué par pressage.

14. Procédé de fabrication d'un composant d'endoprothèse céramique selon la revendication 13, **caractérisé en ce que** le pressage est réalisé de façon uniaxiale ou isostatique.

15. Procédé de fabrication d'un composant d'endoprothèse céramique selon la revendication 13 ou 14, **caractérisé en ce que** le corps moulé pressé est dégazé et fritté.

16. Procédé de fabrication d'un composant d'endoprothèse selon la revendication 13 ou 14, **caractérisé en ce que** le corps moulé fabriqué est dégazé et ensuite soumis à un procédé d'infiltration -comme cela est décrit dans les revendications 7 à 11.

17. Procédé de fabrication d'un élément d'endoprothèse selon l'une quelconque des revendications 7 à 16, **caractérisé en ce qu'**il s'effectue une autre compression du corps moulé fritté par pressage isostatique à température élevée.
